(19) Europäisches Patentamt / European Patent Office / Office européen des brevets

(11) **EP 3 219 324 B1**

(12) **EUROPEAN PATENT SPECIFICATION**

(45) Date of publication and mention
of the grant of the patent:
**26.02.2020 Bulletin 2020/09**

(51) Int Cl.:
*A61K 38/05* [(2006.01)]  *A61K 38/55* [(2006.01)]
*A61P 9/12* [(2006.01)]

(21) Application number: **14899664.8**

(22) Date of filing: **11.08.2014**

(86) International application number:
**PCT/CN2014/084089**

(87) International publication number:
**WO 2016/023150 (18.02.2016 Gazette 2016/07)**

(54) **APPLICATION OF DIPEPTIDE AS ACE ENZYME ACTIVITY INHIBITOR**

VERWENDUNG VON DIPEPTID ALS ACE-ENZYM-AKTIVITÄTSINHIBITOR

APPLICATION D'UN DI-PEPTIDE TENANT LIEU D'INHIBITEUR DE L'ENZYME ECA

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**20.09.2017 Bulletin 2017/38**

(73) Proprietor: **Withyou Biotechnology Co., Ltd.
Guangzhou, Guangdong 501663 (CN)**

(72) Inventors:
• **HE, Xionglei
Guangzhou
Guangdong 510663 (CN)**
• **ZHU, Wei
Guangzhou
Guangdong 510663 (CN)**

(74) Representative: **Wachenhausen & Kollegen
Patentanwälte GbR
Schellingstrasse 29
80799 München (DE)**

(56) References cited:
**EP-A1- 2 161 028      CN-A- 102 432 670
CN-A- 103 736 077**

• **DATABASE WPI Week 200908 Thomson
Scientific, London, GB; AN 2009-B29704
XP002784350, & JP 2008 297208 A (KYOWA
HAKKO KIRIN CO LTD) 11 December 2008
(2008-12-11)**
• **BHUYAN, B.J. ET AL.: 'Effect of Peptide-Based
Captopril Analogues on Angiotensin Converting
Enzyme Activity and Peroxynitrite-Mediated
Tyrosine Nitration' ORGANIC & BIOMOLECULAR
CHEMISTRY vol. 9, 31 December 2011, pages
5185 - 5192, XP055382242**
• **LIN, YINGWU: 'Simulating the Covalent
Modification of Fe (III)-Salophen with Amino Acid
Histidine or Dipeptide CysHis' COMPUTERS AND
APPLIED CHEMISTRY vol. 24, no. 12, 31
December 2007, pages 1607 - 1610, XP008185505**
• 'HMDB ID: HMDB28768, Showing Metabocard for
Cysteinyl-Alanine' **HUMAN METABOLOME
DATABASE 09 February 2013, pages 1 - 6,
XP055382262**
• **BHUYAN, B.J. ET AL.: 'Synthesis,
Characterization and Antioxidant Activity of
Angiotensin Converting Enzyme Inhibitors'
ORGANIC & BIOMOLECULAR CHEMISTRY vol.
9, 07 March 2011, pages 1356 - 1365, XP055382266**
• **LIU, HUAN ET AL.: 'Structural Parameterization
and QSAR of Dipeptide Inhibitor of ACE'
COMPUTERS AND APPLIED CHEMISTRY vol. 22,
no. 8, 28 August 2005, pages 631 - 635**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to a new application of dipeptide, and more particularly, to an application of dipeptide as an ACE enzyme activity inhibitor.

**BACKGROUND OF THE INVENTION**

Angiotensin converting enzyme (ACE) is a zinc metalloproteinase, and a carboxyl dipeptidase, which is one of the important proteases in the renin-angiotensin system. The ACE plays an important role in the regulation of human blood pressure. It removes His-Leu by acting on the terminal of angiotensin I to produce angiotensin II, which allows contraction of arterial smooth muscle, causing rapid rise in blood pressure. An effective method of lowering the blood pressure is inhibiting the ACE activity. Current pharmaceuticals for treatment of high pressure are mostly synthetic chemicals, which have certain adverse effects, such as cough, taste disorders, rashes and other side effects. Thus ACE inhibitory peptides prepared from food-borne proteins as raw material represent an important direction for the development of antihypertensive pharmaceuticals due to the high level of safety, low level of toxic or side effects and other advantages thereof.

Short peptides are easy to prepare, and substantially have no side effects on the human body. Researches show that, a short peptide with specific structure, for example, dipeptide, tripeptide and tetrapeptide, has certain inhibitory effect on the activity of ACE, which makes the short peptide an ACE enzyme inhibitor having a broad development prospect.

LIU Huan, LE Guowei, SHI Yonghui, et al. Structure-activity relationship of angiotensin converting enzyme dipeptide inhibitors [J].Computers and Applied Chemistry, 2006, 22(8):631-635 discloses that a model of structure-activity relationship of angiotensin converting enzyme dipeptide inhibitors is established from the primary structures of peptide chains, by taking molecular electro-negativity edge vector (MEEV) as a parameter, and taking 36 angiotensin converting enzyme dipeptide inhibitors as samples. The rule of "distance of two, five, and seven chemical bond" inhibitory enzyme activity of dipeptide bond is obtained by means of model analysis, said rule means: (1) the carboxyl of the peptide bond forms two-ligand with Zn atom, which is stabilized by the H-bond formed between the N-atom and the carboxyl oxygen of the peptide bond; (2) the five-bond structure unit is formed between the carboxylate radical group acting with Arg (Arginine) positive charged salt bond in the ACE enzyme and the amino group of the second amino acid to play a key role in antihypertensive effect; and (3) the amino group of the peptide bond in the dipeptide inhibitor containing an aromatic amino acid shows trans configuration with the hydroxyl terminal of the benzene ring portion, with seven bonds between them.

LIU Jing, PENG Jianqiu, and GUAN Xiao. Modeling study on quantitative structure-activity relationship of angiotensin converting enzyme inhibitory peptides based on multiple linear regression [J]. Journal Analytical Science, 2012, 28(001):16-22 discloses that a multiple linear regression (MLR) model of structure and activity is established using an amino acid structure describer SVHEHS to characterize dipeptide, tripeptide and tetrapeptide sequences competitively inhibiting Angiotensin Converting Enzyme (ACE), respectively. The correlation coefficient, cross validation correlation coefficient, root mean square error and external validation correlation coefficient are respectively 0.851, 0.781, 0.327, and 0.792 for the ACE inhibitory dipeptide model; respectively 0.805, 0.717, 0.339, and 0.817 for the tripeptide model; and respectively 0.792, 0.553, 0.393, and 0.630 for the tetrapeptide model.

According to Liu Huan. Research on ACE inhibitory peptides in rice [D]. Jiangnan University, 2005 , a model for structure-activity relationship of angiotensin converting enzyme dipeptide inhibitors is established by taking molecular electro-negativity edge vector (MEEV) as a parameter, and taking 36 angiotensin converting enzyme dipeptide inhibitors as samples. The model analysis shows that hydrophobic amino acids, for example, aromatic amino acids and branched-chain amino acids at C-terminal, are key factors affecting ACE inhibitory activity.

The results disclosed in LIU Jing, GUAN Xiao, PENG Jianqiu. QSAR study on ACE inhibitory peptide based on amino acid descriptor SVHEHS [J] Acta Chimica Sinica, 2012, 70(1):83-91 show that the hydrophobicity (X15), electrical property(X17), and stereoscopic feature (X24) of C-terminal amino acid, as well as stereoscopic feature (X12) of N-terminal amino acid of the dipeptide are highly correlated to the activity of the peptide.

The results disclosed in PENG Jianqiu. Research on quantitative structure-activity relationship of ACE inhibitory peptide [D]. University of Shanghai for Science and Technology, 2012 demonstrate a dipeptide model with $R^2$=0.851, RMSE=0.327, $Q^{2LOO}$=0.781, $Q^{2ext}$=0.792, and the hydrophobicity and charge property of the C-terminal amino acid residue and the stereoscopic property of the N-terminal amino acid residue have a relatively strong influence on the activity of an ACE inhibitory dipeptide, particularly, the strong hydrophobicity and weak charge property of the C-terminal amino acid residue have a positive effect on the activity of an ACE inhibitory dipeptide; and the hydrophobicity, electrical property, and stereoscopic property of C-terminal amino acid residue, and the stereoscopic property of N-terminal amino acid residue are highly correlated with the activity of a peptide.

EP 2 161 028 A1 (Unilever NV) discloses a composition comprising peptides. Food product suitable for the prevention or treatment of hypertension comprising one or more peptides wherein the C-terminal amino acid is S, T or D and wherein said peptides are selected from the group consisting of the dipeptides WS, WT, WD, CT and DT.

JP 2008 297208 A (Kyowa Hakko Kirin Co Ltd) discloses an angiotensin I-converting enzyme inhibitor. This angiotensin I-converting enzyme inhibitor contains one or more dipeptides selected from the group consisting of cysteinylglycine, thyrosinylisoleusine, phenylalanylmethionine, 2-aminobutyric acid-phenylalanine and hystidyltryptophan and their salts, as an active ingredient.

Effect of Peptide-Based Captopril Analogues on Angiotensin Converting Enzyme Activity and Peroxynitrite-Mediated Tyrosine Nitration (Bhuyan B.J. et al, Organic & Biomolecular Chemistry, vol. 9, 31 December 2011, pages 5185-5192) discloses that angiotensin converting enzyme (ACE) regulates the blood pressure by converting angiotensin I to angiotensin II and bradykinin to bradykinin 1-7. These two reactions elevate the blood pressure as angiotensin II and bradykinin are vasoconstrictory and vasodilatory hormones, respectively. Therefore, inhibition of ACE is an important strategy for the treatment of hypertension. The natural substrates of ACE, i.e., angiotensin II and bradykinin, contain a Pro-Phe motif near the site of hydrolysis. Therefore, there may be a Pro-Phe binding pocket at the active site of ACE, which may facilitate the substrate binding. In view of this, we have synthesized a series of thiol- and selenol-containing dipeptides and captopril analogues and studied their ACE inhibition activities. This study reveals that both the selenol or thiol moiety and proline residues are essential for ACE inhibition. Although the introduction of a Phe residue to captopril and its selenium analogue considerably reduces the inhibitory effect, there appears to be a Phe binding pocket at the active site of ACE.

Simulating the Covalent Modification of Fe (III)-Salophen with Amino Acid Histidine or Dipeptide CysHis (Lin Yingwu, Computers and Applied Chemistry, vol. 24, no. 12, 31 December 2007, pages 1607-1610) discloses that comparing with the structure of heme group that covalently linked to the polypeptide chain in natural heme proteins, we simulated the covalent modification of artificial Fe(III)-salophen complex with amino acid histidine (His) or dipeptide cysteinyl-histidine (Cys-His), with the intention of providing useful information for design of metalloproteins containing nonnatural prosthetic group. By using molecular mechanics MM+ method, the molecular geometries were optimized, and then the single point calculation was carried out on the obtained structure with minimum energy by using semi-empirical ZINDO/1 method. Results illustrate that, with His serving as the fifth ligand of Fe(III)-salophen, the molecule would be more stable when it was modified covalently, through forming an amide bond with His, by introducing a propionic group rather than a formic group. At the same time, the molecule would be more stable as modification occurred at position a rather than b of Fe(III)-salophen, for both cases of His and Cys-His modification. In addition, of all these modified complexes, the most stable molecule was that being modified with dipeptide Cys-His at position a through a thioether bond that formed between the cysteine residue and an introduced vinyl group. This conformation consists with that found in natural c-type cytochromes. The current study presents the initial theoretical results, which could instruct the successful construction of functional Fe(HI)-salophen complex, and also direct its application in artificial metalloproteins design.

Metabocard for Cysteinyl-Alanine (HMDB28768) discloses that Cysteinyl-Alanine is a dipeptide composed of cysteine and alanine. It is an incomplete breakdown product of protein digestion or protein catabolism. Some dipeptides are known to have physiological or cell-signaling effects although most are simply short-lived intermediates on their way to specific amino acid degradation pathways following further proteolysis. This dipeptide has not yet been identified in human tissues or biofluids and so it is classified as an 'Expected' metabolite.

Synthesis, Characterization and Antioxidant Activity of Angiotensin Converting Enzyme Inhibitors (Bhuyan B.J. et al, Organic & Biomolecular Chemistry, vol. 9, 7 March 2011, pages 1356-1365) discloses that angiotensin converting enzyme (ACE) catalyzes the conversion of angiotensin I (Ang I) to angiotensin II (Ang II). ACE also cleaves the terminal dipeptide of vasodilating hormone bradykinin (a nonapeptide) to inactivate this hormone. Therefore, inhibition of ACE is generally used as one of the methods for the treatment of hypertension. 'Oxidative stress' is another disease state caused by an imbalance in the production of oxidants and antioxidants. A number of studies suggest that hypertension and oxidative stress are interdependent. Therefore, ACE inhibitors having antioxidant property are considered beneficial for the treatment of hypertension. As selenium compounds are known to exhibit better antioxidant behavior than their sulfur analogues, we have synthesized a number of selenium analogues of captopril, an ACE inhibitor used as an antihypertensive drug. The selenium analogues of captopril not only inhibit ACE activity but also effectively scavenge peroxynitrite, a strong oxidant found in vivo.

CN 102 432 670 A (Zhejiang Academy of Agricultural Sciences) discloses silkworm chrysalis protein source dipeptide SS discloses that an amino acid sequence of the silkworm chrysalis protein source dipeptide SS is Ser-Ser. The silkworm chrysalis protein source dipeptide SS can be used as an angiotensin converting enzyme (ACE) inhibitory peptide.

CN 103 736 077 A (Zhejiang Academy of Agricultural Sciences) discloses a dipeptide ES with double functions of lowering blood pressure and lowering blood fat and application thereof. The invention belongs to the technical field of biology, and particularly relates to a dipeptide capable of being combined with angiotensin converting enzyme to inhibit activity of the angiotensin converting enzyme and inhibit activity of 3-hydroxy-3-methylglutaryl coenzyme A (HMG-CoA) reductase. The invention particularly discloses a dipeptide ES with double functions of lowering blood pressure and lowering blood fat, wherein the amino acid sequence of the dipeptide ES is Glu-Ser. The invention also discloses application of the dipeptide ES with double functions of lowering blood pressure and lowering blood fat in preparing ACE (angiotensin converting enzyme) inhibitory peptide and/or HMG-CoA reductase inhibitory peptide.

In prior art, short peptides inhibiting ACE activity are researched from multiple perspectives in an attempt to determine the relationship between the structure of a short peptide and the ACE inhibitory activity. However, the results of the existing researches have limitations in that the accuracy of the predicted results is not high, and no dipeptide with high ACE inhibitory activity is found.

Developing a dipeptide with a high inhibitory activity is of great practical significance.

## SUMMARY OF THE INVENTION

The object of the present invention is to provide an application of a dipeptide as an ACE enzyme activity inhibitor.

The above object is achieved by the technical solution of the present invention as follows.

According to the invention, virtual screening is performed on 400 dipeptides for ACE inhibitory effect based on determined ACE enzyme crystal structure using a self-developed software and a molecular docking method, and experiments are then conducted to verify the ACE inhibitory activities of the dipeptides obtained by virtual screening. It is found that a dipeptide has a relatively improved ACE inhibitory activity when the N-terminal of the dipeptide is a cysteine; and the dipeptide has even better ACE inhibitory activity when the C-terminal amino acid of the dipeptide is a basic amino acid or an aliphatic amino acid; particularly, the dipeptide has best ACE inhibitory activity when the basic amino acid is H or K, or the aliphatic amino acid is A or I.

The amino acid in the dipeptide is in L-form or D-form, and at least one amino acid in the dipeptide may be optionally modified with a group which can improve the stability of the dipeptide *in vivo.*

A dipeptide for inhibition of ACE enzyme activity is provided, wherein the N-terminal of the dipeptide is a cysteine, the amino acid in the dipeptide is in L-form or D-form, and at least one amino acid in the dipeptide may be optionally modified with a group which can improve the stability of the dipeptide *in vivo.*

The C-terminal amino acid of the dipeptide is a basic amino acid or an aliphatic amino and in particular the basic amino acid is H or K, the aliphatic amino acid is A or I.

The present invention has the advantageous effects as follows:

The dipeptides of the present invention can inhibit the activity of ACE greatly, with an ACE inhibition rate of more than 30% at the concentration of $20\mu g/ml$. The inhibitory activities thereof are far beyond those of existing dipeptides. Thus, the dipeptides of the present invention have great potential for further development.

## BRIEF DESCRIPTION OF THE DRAWINGS

Fig. 1 is the kinetic curves with different amounts of ACE enzyme;
Fig. 2 is a relation curve of slope of linear segment of the kinetic curve vs enzyme activity; and
Fig. 3 is an ACE inhibition curve with Captopril.

## DETAILED DESCRIPTION OF THE EMBODIMENTS

The technical solution of the present invention is further described hereinafter with reference to experiments.

### Determination of ACE enzyme activity

*Kinetic curves with different amounts of ACE enzyme*

Preparation for the reaction is made according to the following table:

| No. | 1 | 2 | 3 | 4 | 5 | 6 |
|---|---|---|---|---|---|---|
| FAPGG reagent ($\mu$ l) | 200 | 200 | 200 | 200 | 200 | 200 |
| 115u/L ACE($\mu$ l) | 20 | 16 | 12 | 8 | 4 | 0 |

(continued)

| No. | 1 | 2 | 3 | 4 | 5 | 6 |
|---|---|---|---|---|---|---|
| Ultrapure water ($\mu$l) | 20 | 34 | 28 | 32 | 36 | 40 |
| Note: enzyme activity | 115u/L | 92u/L | 69u/L | 43u/L | 23 u/L | 0 u/L |

The substances are mixed and placed in a SpectraMax microplate reader to determine change of absorbance value with 340nm as a main wavelength and 405 nm as a reference wavelength at 37°C, and continuously monitored for 1 hour.

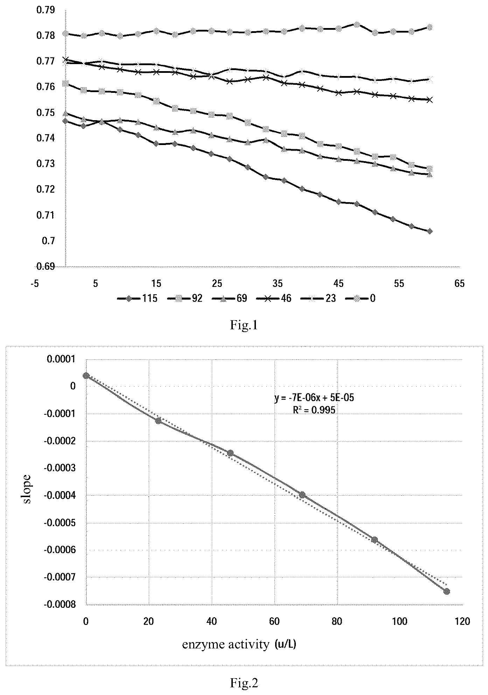

The kinetic curves with different amounts of ACE enzyme are shown in Fig. 1, and the relation curve of slope of linear segment of the kinetic curve vs the enzyme activity is shown in Fig. 2. The results show that, slope of linear segment of the kinetic curve and the ACE enzyme activity are in a linear relation, with a regression equation $y=-7*10^{-6}x+5*10^{-5}$, $R^2=0.995$.

### Inhibitory effect of different concentrations of Captopril on ACE

Captopril is prepared with ultrapure water in 2mg/mL, and then diluted with ultrapure water in 10-time proportion (till $10^{-8}$).

Preparation for the reaction system is shown in the following table:

| No. | 1 | 2 | 3 | 4 | 5 | 6 |
|---|---|---|---|---|---|---|
| FAPGG reagent ($\mu$l) | 200 | 200 | 200 | 200 | 200 | 200 |
| 115u/LACE ($\mu$l) | 20 | 20 | 20 | 20 | 20 | 20 |
| 2$\mu$g/L Captopril ($\mu$l) | 20 | 16 | 12 | 8 | 4 | 0 |
| Ultrapure water ($\mu$l) | 0 | 4 | 8 | 12 | 16 | 20 |
|  |  |  |  |  |  |  |
| FAPGG reagent ($\mu$l) | 200 | 200 | 200 | 200 | 200 | 200 |
| 115u/L ACE ($\mu$l) | 20 | 20 | 20 | 20 | 20 | 20 |
| 0.2$\mu$g/L Captopril ($\mu$l) | 20 | 16 | 12 | 8 | 4 | 0 |
| Ultrapure water ($\mu$l) | 0 | 4 | 8 | 12 | 16 | 20 |

The substances are mixed and placed in a SpectraMax microplate reader to determine change of absorbance value with 340nm as a main wavelength and 405 nm as a reference wavelength at 37°C, and continuously monitored for 1 hour.

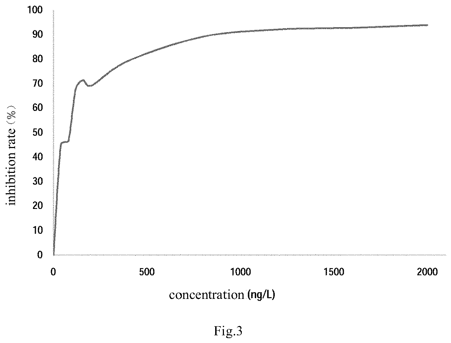

The inhibition curve with Captopril is shown in Fig. 3. It can be seen from Fig. 3 that, viewed from the overall trend, the inhibition rate is decreased as the concentration of Captopril decreases. Since the concentrations are in linear in the experiment, the variation range of the inhibition rate is reduced.

### Inhibitory effect of dipeptides on ACE

20$\mu$g/mL stock solution is prepared by dissolving synthetic dipeptide samples in ultrapure water. The stock solution is then diluted to a 20$\mu$g/mL sample as test sample.

| No. | 1 | 2 | 3 | 4 | 5 | 6 |
|---|---|---|---|---|---|---|
| FAPGG reagent($\mu$l) | 200 | 200 | 200 | 200 | 200 | 200 |
| 115u/LACE($\mu$l) | 20 | 20 | 20 | 20 | 20 | - |
| 20$\mu$g/mL dipeptide ($\mu$l) | 20 | - | - | - | - | - |
| Captopril ($\mu$l) | - | 4 | 20 | 20 | - | - |
| Ultrapure water ($\mu$l) | 0 | 16 | 0 | 0 | 20 | 40 |

The substances are mixed and placed in a SpectraMax microplate reader to determine change of absorbance value with 340nm as a main wavelength and 405 nm as a reference wavelength at 37°C, and continuously monitored for 1 hour. The slopes of linear segment of the enzymatic kinetic curve are calculated, and the inhibition rates of the samples are calculated according to formula (1);

$$\text{Inhibition rate (\%)} = \frac{115 - E}{115} \times 100$$

Wherein, $E = \dfrac{S_s - S_b}{S_p - S_b} \times 115$

E-ACE enzyme activity in a sample well

$S_s$-slope of linear segment of ACE kinetic curve for a sample well

$S_p$-slope of linear segment of ACE kinetic curve for a well without inhibitor

$S_b$-slope of linear segment of ACE kinetic curve for a blank well

The experimental results are shown in the following table:

| No. | Dipeptide | Inhibition rate (%) |
|---|---|---|
| 3 | FE | 6.18 |
| 5 | KW | 16.42 |
| 9 | IF | 18.35 |
| 11 | KY | 11.54 |
| 13 | AY | 8.18 |
| 15 | KP | 7.88 |
| 19 | WL | 7.27 |
| 20 | KA | 6.45 |
| 22 | AG | 3,08 |
| 25 | **CA** | **84.38** |
| 28 | **CH** | **70.79** |
| 29 | **CI** | **67.52** |
| 30 | **CK** | **38.39** |
| 38 | DE | 15.16 |
| 47 | EV | 14.26 |
| 48 | EW | 9.91 |
| 49 | FD | 5.05 |
| 50 | FH | 10.06 |
| 51 | FI | 9.58 |
| 55 | FQ | 10.96 |
| 57 | FT | 1.92 |
| 58 | FW | 3.16 |
| 59 | GC | 0.12 |

(continued)

| No. | Dipeptide | Inhibition rate (%) |
|---|---|---|
| 64 | GT | -3.20 |
| 65 | HC | 5.06 |
| 66 | HD | 10.98 |
| 67 | HE | 9.95 |
|  | Cap1 | 86.95 |
|  | Cap2 | 72.84 |
|  | Cap3 | 35.84 |

It can be seen from the table that when the N-terminal of a dipeptide is a cysteine, the ACE inhibition rate of the dipeptide is significantly higher than those of other dipeptides; and more particularly, when the dipeptide is CA, CH, CI or CK, the inhibition rate is even higher.

## Claims

**1.** A dipeptide for use in lowing blood pressure, wherein the N-terminal of the dipeptide is a cysteine, the C-terminal amino acid of the dipeptide is a basic amino acid or an aliphatic amino acid, the basic amino acid is H or K, the aliphatic amino acid is A or I, each amino acid in the dipeptide is in L-form or D-form.

## Patentansprüche

**1.** Dipeptid zur Verwendung bei der Senkung des Blutdrucks, wobei das N-terminale Ende des Dipeptids ein Cystein ist, die C-terminale Aminosäure des Dipeptids eine basische Aminosäure oder eine aliphatische Aminosäure ist, die basische Aminosäure H oder K ist, die aliphatische Aminosäure A oder I ist, jede Aminosäure in dem Dipeptid in der L- oder D-Form vorliegt.

## Revendications

**1.** Dipeptide pour une utilisation dans l'abaissement de la pression sanguine, le N-terminal du dipeptide étant une cystéine, l'acide aminé C-terminal du dipeptide étant un acide aminé basique ou un acide aminé aliphatique, l'acide aminé basique étant H ou K, l'acide aminé aliphatique étant A ou I, chaque acide aminé dans le dipeptide se trouvant sous forme L ou sous forme D.

Fig.1

Fig.2

Fig.3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2161028 A1
- JP 2008297208 A

- CN 102432670 A
- CN 103736077 A

### Non-patent literature cited in the description

- **LIU HUAN ; LE GUOWEI ; SHI YONGHUI et al.** Structure-activity relationship of angiotensin converting enzyme dipeptide inhibitors [J. *Computers and Applied Chemistry,* 2006, vol. 22 (8), 631-635
- **LIU JING ; PENG JIANQIU ; GUAN XIAO.** Modeling study on quantitative structure-activity relationship of angiotensin converting enzyme inhibitory peptides based on multiple linear regression [J. *Journal Analytical Science,* 2012, vol. 28 (001), 16-22
- **LIU HUAN.** Research on ACE inhibitory peptides in rice [D. Jiangnan University, 2005

- **LIU JING ; GUAN XIAO ; PENG JIANQIU.** QSAR study on ACE inhibitory peptide based on amino acid descriptor SVHEHS [J. *Acta Chimica Sinica,* 2012, vol. 70 (1), 83-91
- **PENG JIANQIU.** Research on quantitative structure-activity relationship of ACE inhibitory peptide [D. University of Shanghai for Science and Technology, 2012
- **BHUYAN B.J. et al.** *Organic & Biomolecular Chemistry,* 31 December 2011, vol. 9, 5185-5192
- **LIN YINGWU.** *Computers and Applied Chemistry,* 31 December 2007, vol. 24 (12), 1607-1610
- **BHUYAN B.J. et al.** *Organic & Biomolecular Chemistry,* 07 March 2011, vol. 9, 1356-1365